# FASCICULE DE BREVET EUROPEEN

(11) **EP 2 816 947 B1**
(45) Date de publication et mention de la délivrance du brevet: **06.04.2022**
(21) Numéro de dépôt: 13704098.6
(22) Date de dépôt: 12.02.2013
(51) Int. Cl.: A61B 1/04, A61B 1/055, A61B 1/00, G02B 23/24

(54) **DISPOSITIF ENDOSCOPIQUE DESTINÉ NOTAMMENT À UN USAGE MÉDICAL**
ENDOSKOPISCHE VORRICHTUNG, INSBESONDERE FÜR EINE MEDIZINISCHE ANWENDUNG
ENDOSCOPIC DEVICE INTENDED, IN PARTICULAR, FOR A MEDICAL USAGE

(30) Priorité: 24.02.2012 FR 1251711
(43) Date de publication de la demande: 31.12.2014
(73) Titulaire: l'Helgoual'ch, Guy, 29200 Brest (FR)
(72) Inventeur: L'HELGOUAL'CH, Guy, F-29200 Brest (FR)
(74) Mandataire: Vidon Brevets & Stratégie
(86) Numéro de dépôt international: PCT/EP2013/052758
(87) Numéro de publication internationale: WO 2013/124184

(56) Documents cités:
- WO-A1-00/54033
- WO-A1-2011/154970
- FR-A1- 2 899 087
- US-A- 5 457 576
- US-A- 5 881 195
- US-A1- 2002 087 047
- US-A1- 2005 182 297
- ROL P ET AL: "OPTICAL PROPERTIES OF MINIATURIZED ENDOSCOPES FOR OPHTHALMIC USE", OPTICAL ENGINEERING, SOC. OF PHOTO-OPTICAL INSTRUMENTATION ENGINEERS, BELLINGHAM, vol. 34, no. 7, 1 July 1995 (1995-07-01), pages 2070-2076, XP000517073, ISSN: 0091-3286, DOI: 10.1117/12.204800

## Description

### 1. DOMAINE DE L'INVENTION

Le domaine de l'invention est celui de l'instrumentation endoscopique destinée à l'exploration et la visualisation de l'intérieur d'organes, de conduits ou de cavités qui sont inaccessibles à l'œil nu.

Plus précisément, l'invention concerne un dispositif endoscopique comprenant une tête de guidage optique destinée à être insérée dans un corps ou une cavité pour visualiser ou magnifier un objet ou un site qui est difficilement accessible à l'œil nu.

L'invention a de nombreuses applications, entre autres dans le domaine de l'imagerie médicale pour l'examen interne d'organes du corps humain à des fins diagnostiques et/ou thérapeutiques (endoscopie opératoire), ou bien dans le domaine industriel pour la visualisation et le contrôle de structures internes de corps creux et de pièces mécaniques.

### 2. ARRIÈRE-PLAN TECHNOLOGIQUE

On s'attache plus particulièrement dans la suite de ce document à décrire la problématique existant dans le domaine de l'imagerie médicale, et plus particulièrement dans le domaine de l'imagerie oculaire (visualisation des structures internes de l'œil) à laquelle ont été confrontés les inventeurs de la présente demande de brevet. L'invention ne se limite bien sûr pas à ce domaine particulier d'application, mais présente un intérêt pour tout dispositif endoscopique (ou endoscope) devant faire face à une problématique proche ou similaire.

Les dispositifs endoscopiques utilisés de nos jours pour examiner la structure de l'œil humain comportent une partie invasive rigide, appelée tête de guidage optique, destinée à pénétrer l'organe d'un patient. Cette tête de guidage optique est formée généralement d'un tube métallique de quelques millimètres de diamètre externe (par exemple un tube en acier inoxydable de 0,9 millimètres de diamètre externe) contenant un système optique composé soit d'une série de lentilles optiques, soit d'un faisceau de fibres optiques agglutinées avec une colle de cohésion.

Typiquement, le faisceau de fibres optiques est connecté à un capteur d'image (comme une caméra vidéo par exemple) au moyen d'un adaptateur, le capteur d'image étant lui-même raccordé à un écran d'affichage. Le faisceau de fibres optiques permet le transport des images de l'objet observé jusqu'au capteur d'image qui, après mise en forme, les retransmet en temps réel à l'écran d'affichage.

Le tube métallique est également muni d'un système d'éclairage par fibre optique permettant de transporter la lumière depuis un boîtier externe muni d'une source lumineuse jusqu'à la zone d'observation.

Un moyen de préhension est solidarisé au tube métallique pour faciliter la manipulation de l'endoscope et le repérage spatial.

Ce type d'endoscope présente néanmoins plusieurs inconvénients.

Pour assurer la rigidité de la tête de guidage optique lors de son introduction dans l'organe du patient, le tube métallique doit présenter une certaine épaisseur, ce qui limite la quantité de lumière atteignant le capteur d'image. La quantité de détails capturés par le capteur d'image étant ainsi limitée, la qualité des images restituées sur l'écran se trouve être relativement dégradée.

En outre, les têtes de guidage optique qu'elles soient composées de lentilles optiques ou de fibres optiques sont techniquement complexes et coûteuses à fabriquer.

En effet, une tête de guidage à lentilles optiques nécessite un assemblage très précis d'une pluralité de lentilles optiques dans un tube métallique de dimensions réduites du fait des exigences de miniaturisation (le diamètre interne du tube pouvant atteindre parfois quelques centaines de micromètres), pour former l'objectif de l'endoscope. Les lentilles sont des composants optiques particulièrement fragiles.

En outre, le câble d'une tête de guidage comporte généralement un faisceau de fibres optiques de grande longueur (pouvant attendre jusqu'à plusieurs mètres) occasionnant des pertes de lumière non négligeable. Le câble de fibres optiques est imposant et ne permet pas un maniement aisé de l'endoscope.

Enfin, pour éviter tout risque d'infection du patient, il est nécessaire de disposer d'un matériel endoscopique aseptisé. À cette fin, les endoscopes sont stérilisés avant chaque usage (comme les opérations de stérilisation à l'autoclave par exemple), conduisant souvent à un vieillissement prématuré des endoscopes. Ceci inclut non seulement la tête de guidage optique en contact direct avec le patient, le moyen de préhension, mais également le câble de fibres optiques, rendant les opérations en autoclave malaisées et source de casse et dégradation du système.

D'autres dispositifs endoscopiques sont connus des documents US 2005/0182297 A1 et WO 2011/154970 A1.

Il existe donc un besoin pour un dispositif endoscopique peu coûteux, simple à utiliser, offrant des garanties en termes de sécurité sanitaire et permettant la restitution d'images de bonne qualité.

### 3. OBJECTIFS DE L'INVENTION

L'invention, dans au moins un mode de réalisation, a notamment pour objectif de pallier ces différents inconvénients de l'état de la technique.

Plus précisément, dans au moins un mode de réalisation de l'invention, un objectif est de fournir une technique qui permette d'obtenir un dispositif endoscopique qui soit de coût réduit et d'une grande simplicité de montage et de fabrication.

Au moins un mode de réalisation de l'invention a également pour objectif de fournir une telle technique qui permette d'augmenter la qualité des images restituées par le dispositif endoscopique comparativement aux images restituées par les dispositifs endoscopiques de l'art antérieur.

En d'autres termes, un des objectifs de cette technique, dans au moins un mode de réalisation, est de rendre plus aisée l'usage de dispositifs endoscopiques dans le cadre par exemple de l'imagerie médicale.

Un autre objectif d'au moins un mode de réalisation de l'invention est de fournir une telle technique qui permette de s'affranchir de la contrainte relative au vieillissement prématuré des composants dû aux opérations de stérilisation.

### 4. EXPOSÉ DE L'INVENTION

L'invention est définie dans la revendication 1. Dans un mode de réalisation particulier de l'invention, il est proposé un dispositif endoscopique comprenant une tête de guidage optique destinée à être insérée, au moins en partie, dans un corps ou une cavité pour visualiser un objet donné inaccessible à l'œil nu, ladite tête de guidage optique étant constituée d'un barreau optique à gradient d'indice de réfraction.

On entend par « barreau optique à gradient d'indice », un composant optique plein (par opposition à un tube) de section quelconque, réalisé dans un matériau dont les caractéristiques mécaniques, géométriques et optiques permettent de réaliser :
- la fonction de tige endoscopique invasive s'introduisant à l'intérieur d'un corps ou d'une cavité inaccessible à l'œil nu ;
- la fonction de guide d'onde optique, permettant le transport des rayons lumineux issus de l'objet à visualiser et la formation d'images dans un plan focal image prédéfini.

Alors que dans les dispositifs endoscopiques de l'art antérieur la tête de guidage optique nécessite deux éléments distincts l'un pour jouer le rôle de partie invasive (tube métallique rigide) et l'autre pour jouer le rôle de guide optique (lentilles optiques ou fibres optiques), la tête de guidage optique selon l'invention fait appel à un seul et même élément pour réaliser simultanément ces deux fonctions.

Le barreau optique bénéficiant d'ores et déjà les caractéristiques mécaniques d'une tige endoscopique invasive, il devient alors inutile de prévoir au niveau de la tête de guidage optique un tube métallique rigide comme dans les endoscopes de l'art antérieurs, et la totalité du diamètre du barreau optique (et donc de la tête de guidage optique) peut être utilisée pour guider la lumière depuis son extrémité distale jusqu'à son extrémité proximale. N'étant plus limité par l'épaisseur du tube métallique, le champ de captation de la tête optique est donc augmenté, ce qui permet de guider une plus grande quantité de lumière au travers de la tête de guidage optique. En outre, grâce au barreau optique, l'utilisation d'un faisceau de fibres optiques de grande longueur ne s'avère plus nécessaire. La solution de l'invention offre donc aux utilisateurs l'avantage de disposer d'un dispositif endoscopique restituant des images de bien meilleure qualité que celles restituées par les dispositifs de l'art antérieur (pas de perte liée à la présence de fibres optiques, ni de limitation du champ de captation due au tube métallique), ce qui facilite l'interprétation des images.

Les opérations complexe de montage de lentilles optiques ou de fibres optiques dans un tube métallique pour obtenir une tête de guidage optique ne s'avérant plus nécessaires, le coût de montage et de fabrication d'un tel dispositif s'en trouve donc diminué.

Ainsi, de manière avantageuse, le matériau et le profil du gradient d'indice sont choisis de sorte que le barreau optique remplisse les deux fonctions précitées.

Selon une caractéristique avantageuse, le dispositif endoscopique comprend un capteur d'image et le barreau optique à gradient d'indice de réfraction est tel qu'il comprend une extrémité proximale et une extrémité distale chacune traitée de façon à former une image sur le capteur d'image à partir de rayons lumineux issus de l'objet à visualiser.

L'utilisation d'une série de lentilles optiques ou d'un faisceau de fibres optiques pour former l'image de l'objet à visualiser sur le capteur d'image ne s'avère donc plus nécessaire puisque la forme des extrémités du barreau optique associée au gradient d'indice de réfraction remplit déjà cette fonction.

De manière avantageuse, au moins l'une des dites extrémité proximale et distale appartient à la liste comprenant :
- une extrémité plane ;
- une extrémité asphérique ;
- une extrémité en Fresnel diffractif.

Cette liste n'est pas exhaustive.

En particulier, une lentille asphérique a pour avantage d'éviter l'apparition d'aberration optique.

Avantageusement, le dispositif endoscopique comprend une lentille optique d'élargissement de champ de captation agencée sur l'extrémité distale du barreau optique.

Ceci permet d'élargir le champ de captation du barreau optique, paramètre important notamment dans le cadre d'opérations chirurgicales oculaires par exemple, qui nécessitent généralement l'emploi d'une tête de guidage optique de faibles diamètres (0,9mm par exemple).

Selon l'invention, le barreau optique à gradient d'indice comprend au moins un rail longitudinal apte à recevoir une fibre optique d'éclairage pour éclairer l'objet à visualiser à proximité de l'extrémité distale du barreau optique.

La présence d'une fibre optique d'éclairage dans le rail longitudinal provoque l'apparition d'une tâche périphérique dans l'image circulaire, pouvant servir de repère d'orientation de l'image pour l'utilisateur.

Selon une première variante de réalisation, le dispositif comprend un moyen de préhension de sorte que ledit moyen de préhension et le barreau optique à gradient d'indice formant un ensemble monobloc.

Cette variante de réalisation offre ainsi, à tout utilisateur, l'avantage d'un dispositif endoscopique « clé en main », simple d'utilisation, et pouvant être à usage unique. En effet, l'ensemble monobloc peut être conçu dans un matériau permettant un usage unique dudit ensemble monobloc.

Par usage unique, on entend que le mode de fabrication de l'ensemble monobloc est adapté pour que l'ensemble monobloc puisse être utilisé une seule fois. L'emploi d'un dispositif endoscopique à usage unique permet notamment de garantir une bonne hygiène dudit dispositif.

Selon une deuxième variante de réalisation, le dispositif comprend un moyen de préhension accouplé mécaniquement avec le barreau optique à gradient d'indice via des moyens d'accouplement mécanique réversible.

Ce mode de réalisation offre ainsi, à tout utilisateur, la possibilité de monter ou de démonter le dispositif endoscopique en deux parties distinctes grâce à la présence des moyens d'accouplement mécanique réversible.

On peut prévoir de mettre en œuvre des têtes de guidage optiques interchangeables, par exemple dans le cadre d'un usage particulier nécessitant un changement de diamètre du barreau optique ou encore de l'optique de prise de vue inhérent au barreau optique.

D'autre part, un tel mode de réalisation permet à l'utilisateur de stériliser le moyen de préhension, le barreau optique (démontable du moyen de préhension) étant l'élément sensible aux hautes températures. Ceci permet donc un usage unique de la tête de guidage optique, ce qui garantit une bonne hygiène de celui-ci.

Les moyens d'accouplement mécanique réversible appartiennent au groupe comprenant :
- moyens d'accouplement par clipsage ;
- moyens d'accouplement par vissage ;
- moyens d'accouplement par emboîtement ;
- moyens d'accouplement à liaisons mâle et femelle ;
- moyens d'accouplement à baïonnette.

Cette liste n'est pas exhaustive.

Selon une caractéristique avantageuse, le capteur d'image est un capteur d'image embarqué dans le moyen de préhension.

Les utilisateurs disposent de cette façon un dispositif endoscopique avec capteur embarqué. Il peut s'avérer avantageux de prévoir un port de communication sans-fil pour transmettre les images de l'objet visualisé vers un écran d'affichage. En l'absence de liaison filaire, les manipulations chirurgicales sont rendues plus aisées.

Avantageusement, le dispositif comprend une optique d'adaptation de focale agencée à proximité du capteur d'image embarqué, entre le barreau optique à gradient d'indice et le capteur d'image embarqué.

Ceci permet d'imager l'objet à visualiser sur le capteur d'image avec davantage de précision.

L'optique d'adaptation de focale appartient au groupe comprenant :
- une lentille optique à focale fixe ;
- une série de lentilles amovibles à focale variable ;
- une optique adaptative à base de liquide ou de cristal liquide.

L'invention prévoit ainsi la possibilité d'implémenter soit une lentille fixe, soit une série de lentilles amovibles, soit une optique adaptative devant le capteur d'image. La lentille fixe permet d'ajuster la distance focale du système optique que constitue le barreau optique. L'optique adaptative permet de mettre en œuvre, par modulation d'un indice de réfraction du liquide ou du cristal liquide :
- un mécanisme de mise au point variable de la netteté de l'image sur le plan focal du capteur d'image ;
- un mécanisme d'ajustement de distance focale variable.

Ainsi, le dispositif endoscopique offre ici la possibilité d'adapter l'optique de prise de vue de la tête de guidage optique.

Avantageusement, le moyen de préhension comprend en outre au moins une source de lumière embarquée et/ou une alimentation électrique autonome.

N'ayant plus besoin de prévoir d'alimentation lumineuse et/ou d'alimentation électrique pour rendre le dispositif endoscopique opérationnel, le maniement du dispositif endoscopique en est d'autant plus facilité.

Selon l'invention, le barreau optique à gradient d'indice est réalisé en verre organique. Selon d'autres façons avantageuses et n'appartenant pas à l'invention, le barreau optique peut être réalisé dans un matériau optiquement transparent appartenant au groupe comprenant :
- polyméthacrylate de méthyle (PMMA) ;
- polycarbornate ;
- verre.

Il convient de noter que cette liste n'est pas exhaustive.

De façon avantageuse, le barreau optique à gradient d'indice est recouvert d'un film de protection opaque.

Un tel film de protection permet d'éviter que des rayons lumineux parasites (« bruit ») ne viennent s'ajouter aux rayons lumineux issus de l'objet à visualiser, par exemple lors d'un contact du barreau optique avec des tissus mouillés, ce qui aurait pour conséquence la dégradation de la qualité des images restituées.

### 5. LISTE DES FIGURES

D'autres caractéristiques et avantages de l'invention apparaîtront à la lecture de la description suivante, donnée à titre d'exemple indicatif et non limitatif, et des dessins annexés, dans lesquels :
- la figure 1 présente une vue d'ensemble d'un dispositif endoscopique selon un premier mode de réalisation de l'invention ;
- la figure 2 présente une vue d'ensemble d'un dispositif endoscopique selon un deuxième mode de réalisation de l'invention.

### 6. DESCRIPTION DÉTAILLÉE

Sur toutes les figures du présent document, les éléments identiques sont désignés par une même référence numérique.

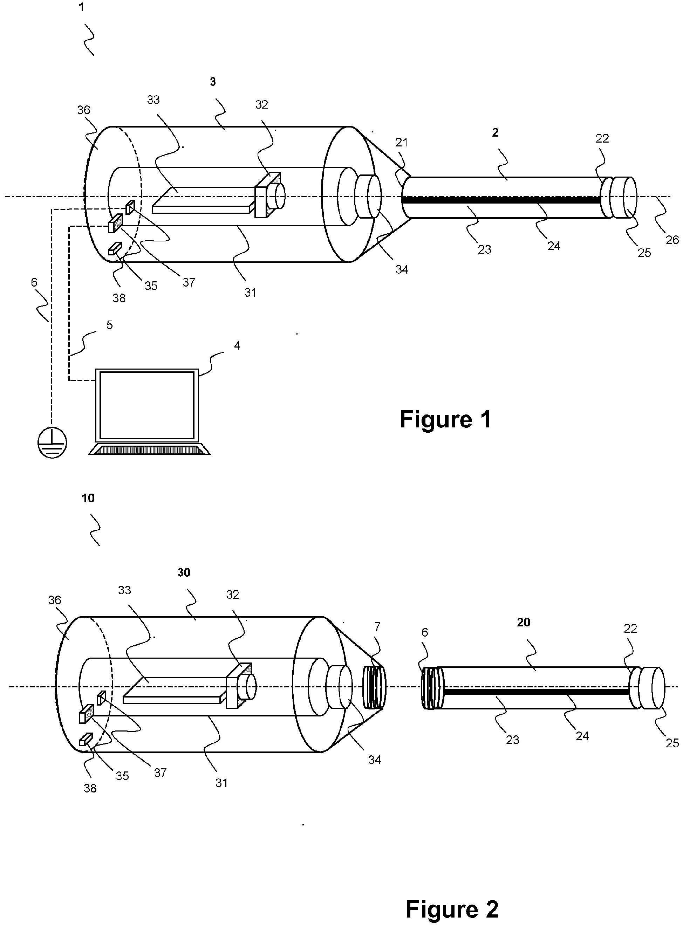

La **figure 1** représente une vue d'ensemble d'un dispositif endoscopique 1 selon un premier mode de réalisation de l'invention. Le dispositif endoscopique comporte une poignée de préhension 3, en forme de manchon, et une tête de guidage optique 2.

La tête de guidage optique 2 est constituée d'un barreau optique, de section circulaire, réalisé dans un matériau à gradient d'indice de réfraction radial. On entend par matériau à gradient d'indice de réfraction radial, un matériau présentant un indice de réfraction non homogène décroissant suivant la distance depuis l'axe optique 26 du barreau jusqu'à sa périphérie extérieure de celui-ci. Ceci permet d'obtenir un effet de convergence optique des rayons lumineux entrant dans le barreau optique 2.

À titre d'exemple purement illustratif, le barreau optique 2 est réalisé en PMMA (pour « polyméthacrylate de méthyle ») avec un gradient d'indice de réfraction ayant un profil polynomial d'ordre 4 et un indice de réfraction de cœur de 1,49. Il est de forme cylindrique et présente par exemple une longueur de 35 millimètres et un diamètre de 0,9 millimètres environ. D'autres dimensions, profils d'indice et matériaux sont bien entendu possibles sans sortir le cadre de l'invention. Les opérations chirurgicales oculaires, par exemple, nécessitent généralement l'emploi d'une tête de guidage optique de faibles diamètres, pouvant être compris entre 0,5mm et 1,5mm, la longueur de la tête optique devant être alors adaptée de façon à remplir la fonction de guide d'onde optique.

Le profil de gradient d'indice du barreau optique 2 peut être obtenu à titres d'exemples par dopage ionique, par étirage, par refroidissement spécifique d'un matériau optique ou toute autre technique bien connue de l'Homme du Métier, comme celles couramment utilisées pour la fabrication de fibres optiques télécoms par exemple. L'extrémité proximale 21 et l'extrémité distale 22 sont usinées chacune sur leur surface extérieure de manière à obtenir deux profils distincts, de sorte que les rayons lumineux émanant de l'objet visualisé traversent le barreau optique à gradient d'indice 2 depuis l'extrémité distale 22 et en forment une image dans le plan focal du capteur d'image 32. Les extrémités proximale 21 et distale 22 sont donc traitées de manière à participer, avec le gradient d'indice de réfraction du barreau, à l'effet de convergence optique des rayons lumineux pour former les images dans le plan focal du capteur d'image 32.

Dans le cas où le barreau optique 2 est obtenu par moulage, le profil des surfaces des proximale 21 et distale proximale 21, ainsi que les dimensions du barreau, sont définies par l'empreinte du moule. Il suffit en effet d'adapter les dimensions du moule pour obtenir les profils de surface et les dimensions souhaités. Grâce à ses caractéristiques optiques et mécaniques particulières, le barreau optique 2 selon l'invention constitue un unique élément qui agit comme un guide d'onde optique, essentiel pour transporter les rayons lumineux issus de l'objet visualisé jusqu'au capteur d'image 32, et comme une tige invasive suffisamment rigide pour pouvoir être introduit dans l'œil d'un patient par exemple, ou tout autre organe, sans se casser. Quand les endoscopes de l'art antérieur font appels à deux éléments distincts pour réaliser une tête optique, l'un pour remplir la fonction de guide d'onde optique (fibres optiques ou lentilles optiques) et l'autre de tige invasive (tube métallique), le dispositif endoscopique 1 de l'invention nécessite un seul élément - le barreau optique à gradient d'indice 2 - pour remplir ces deux fonctions.

Il convient de noter que l'Homme du Métier adaptera le matériau ainsi que le profil de gradient d'indice à utiliser pour le barreau optique 2 de sorte que les conditions précitées soient respectées. D'une manière générale, le barreau optique possède des caractéristiques optiques suffisantes pour pouvoir imager l'objet visualisé sur un capteur d'image (et remplir ainsi la fonction de guide d'onde) et être suffisamment rigide pour pouvoir être inséré dans un organe (et remplir ainsi la fonction de tige invasive).

Selon l'invention, le barreau optique est constitué de verre organique.

Les exemples suivants, donnés à titre d'exemples purement illustratifs, non limitatifs, et ne formant pas partie de l'invention, rendent compte de quelques uns des matériaux dont peut être constitué le barreau optique : polycarbonate, verre minéral, etc. Le barreau optique 2 selon l'invention comprend en outre un rail de longitudinal 23 permettant de venir loger une fibre optique d'éclairage 24 éclairant l'objet à visualiser en un point proche de l'extrémité distale 22. La fibre optique 24 est alimentée en lumière depuis l'extérieur à l'aide d'un boîtier d'alimentation (non illustré), celui-ci étant équipé par exemple d'une lampe en tungstène.

La poignée de préhension 3 est de dimensions adaptées pour une prise en main aisée du dispositif endoscopique 1 et pour une intégration d'éléments de capture vidéo. Plus précisément, la poignée de préhension 3 comprend une caméra vidéo embarquée 31, celle-ci comportant d'une part un capteur d'image 32, par exemple de type CCD (pour « charged couple device ») en technologie CMOS (pour « Complémentary Métal Oxyde Semiconductor »), et d'autre part une carte électronique 33 pour la gestion des images capturées par le capteur d'image et la commande de certaines fonctionnalités du dispositif endoscopique 1.

Les rayons lumineux provenant de l'objet filmé viennent illuminer le capteur d'image 32 via le barreau optique 2 qui remplit la fonction d'objectif optique. Le capteur d'image 32 convertit les rayons lumineux (photons) qu'il reçoit en signaux électriques (charges électriques). Pour chaque pixel illuminé, un signal électrique est numérisé, puis analysé par un circuit intégré (non illustré) dans la carte électronique. Chaque image capturée par le capteur d'image 32 est ensuite envoyée, sous forme d'un signal vidéo d'affichage, vers un écran d'affichage 4.

Le dispositif endoscopique 1 peut être équipé d'une interface homme/machine, par exemple située sur la face arrière 36 de la poignée de préhension 3, comprenant :
- un commutateur d'alimentation 37, par exemple de type marche/arrête, pour mettre le dispositif endoscopique 1 sous tension ou hors tension ;
- un port de communication filaire 35, par exemple de type USB (pour « Universal Serial Bus »), destiné à recevoir un câble 5 assurant la sortie du signal vidéo d'affichage vers un écran d'affichage 4 ;
- un port d'alimentation 38 pour l'alimentation électrique du dispositif endoscopique 1, via un câble d'alimentation 6.

Dans une variante de réalisation, on pourrait prévoir l'implémentation, sur l'interface homme/machine 36, d'un unique port assurant à la fois la sortie du signal vidéo d'affichage et l'alimentation électrique du dispositif endoscopique 1.

Selon une autre variante de réalisation, l'alimentation électrique du dispositif endoscopique 1 pourrait être assurée au moyen d'une alimentation électrique autonome telle qu'une batterie ou des piles rechargeables par exemple. Dans ce cas, la poignée de préhension 3 comprend un logement (non illustré) de dimension adaptée à l'intégration de ladite batterie ou desdites piles rechargeables. La sortie du signal vidéo d'affichage pourrait être assurée dans ce cas à l'aide de moyens de transmission sans-fil (non illustrés), par exemple de type Wi-Fi ou 4G, intégré au niveau de l'interface homme/machine 36, et destiné à communiquer avec des moyens de réception sans-fil intégrés à l'écran d'affichage 4. La poignée de préhension peut comprendre avantageusement un autre logement (non illustré) de dimension adaptée pour intégrer une lampe d'éclairage, par exemple de type LED (pour « Light Emitting Diode »), destinée à alimenter la fibre optique d'éclairage 24 en lumière. Ainsi, dans cette variante de réalisation, aucun câble d'alimentation électrique, d'alimentation en lumière ou de transmission de signaux vidéo d'affichage, ne s'avère désormais nécessaire, ce qui rend le dispositif endoscopique 1 d'une part beaucoup plus maniable, et d'autre part totalement autonome.

Le mode de réalisation décrit ici à la figure 1 illustre un dispositif endoscopique intégrant une caméra vidéo embarquée 31. Il est clair de nombreux autres modes de réalisation de l'invention peuvent être envisagés. On peut notamment prévoir une poignée de préhension disposant d'un logement longitudinal (non illustré) dont les dimensions sont adaptées pour loger une caméra vidéo externe au sein du dispositif endoscopique 1. Ceci présente l'avantage de pouvoir utiliser une caméra plus haut de gamme, et de ne jeter, après utilisation, que l'ensemble monobloc comprenant le barreau optique 2 et la poignée de préhension 3, la caméra vidéo pouvant être réutilisée ultérieurement pour d'autres interventions. Dans ce cas, la caméra vidéo devra disposer elle-même d'une interface homme/machine comme l'interface 36 de la poignée de préhension décrite plus haut (les variantes d'implémentation de cette interface, détaillées plus haut, pouvant également s'appliquer à ce mode de réalisation).

De manière optionnelle, l'interface homme/machine 36 peut être équipé en outre d'un écran d'affichage numérique sur lequel s'affichent les images de l'objet visualisé avec le dispositif endoscopique 1.

La poignée de préhension 3 comprend en outre une optique d'adaptation de focale 34 placée devant le capteur d'image embarqué 32, entre le barreau optique 2 et le capteur d'image embarqué 32. Selon une implémentation particulière, cette optique d'adaptation de focale 34 peut faire partie intégrante de la caméra vidéo 31. Cette optique vise à imager avec davantage de précision, l'objet filmé avec le dispositif endoscopique, sur le capteur d'image. Elle peut être une lentille fixe, une série de lentilles mécaniquement amovibles, ou une optique adaptative telle qu'une lentille liquide ou à cristal liquide. La lentille fixe permet d'ajuster la distance focale du système optique constitué par le barreau optique 2. L'optique adaptative permet, sous l'effet d'au moins un signal électrique de commande, d'ajuster la distance focale du système optique et/ou la mise au point de la netteté des images, par modulation d'un indice de réfraction du liquide ou du cristal liquide.

Dans le mode de réalisation présenté ici, le barreau optique 2 et la poignée de préhension 3 sont réalisés dans la même matière et forment un ensemble monobloc. Cet ensemble monobloc peut être obtenu par diverses méthodes de fabrication largement décrites dans la littérature. À titre d'exemple purement illustratif, l'ensemble monobloc 2, 3 peut être réalisé en PMMA selon une technique standard de moulage permettant une fabrication en environnement industriel de volume.

Un dispositif endoscopique monobloc peut être également réalisé en solidarisant de manière définitive le barreau optique 2 et la poignée de préhension 3, le barreau optique 2 étant réalisé selon une technique standard de fabrication similaire à celle utilisée pour l'élaboration des fibres optiques et la poignée de préhension 3 selon une technique standard de moulage. Bien entendu, la poignée de préhension 3 peut être réalisée dans ce cas dans un matériau qui est différent du matériau utilisé pour la fabrication du barreau optique 2.

Le PMMA étant un matériau bas coût, l'invention permet d'obtenir un dispositif endoscopique monobloc de coût réduit et d'une grande simplicité de fabrication. Ainsi, lorsque celui-ci intègre des composants bas coûts, comme la caméra vidéo embarquée par exemple, l'invention permet un usage unique du dispositif endoscopique, ce qui peut s'avérer particulièrement avantageux dans le cadre d'interventions chirurgicales notamment, après chaque utilisation, le dispositif endoscopique pouvant être jeté, évitant ainsi toutes problématiques liées aux opérations de stérilisation.

Enfin, le barreau optique 2 comprend ici une lentille optique d'élargissement de champ de captation 25 agencée sur son extrémité distale 22, pour élargir le champ de captation du barreau optique 2. Le barreau optique 2 peut comprend en outre un film de protection (non illustré sur la figure) agencé sur la périphérie extérieure de celui-ci, pour éviter la capture de rayons lumineux parasites (« bruit ») par le capteur d'image 32.

Il peut s'avérer également avantageux de disposer d'un dispositif endoscopique montable/démontable, comme illustré ci-après en relation avec la **figure 2****.**

La figure 2 présente une vue d'ensemble d'un mode de réalisation particulier du dispositif endoscopique 10 dans lequel celui-ci est démonté. Il comprend comme pour le dispositif 1 de la figure 1 un barreau optique 20, formant tête de guidage optique, et une poignée de préhension 30 sous forme d'un manchon. Ce mode de réalisation permet avantageusement de mettre en œuvre un dispositif endoscopique à têtes optiques interchangeables.

Par exemple, une même opération chirurgicale peut nécessiter l'utilisation d'une pluralité de barreaux optiques de diamètres différents ; un barreau optique d'un diamètre donné peut très bien être adapté pour une étape particulière et inadapté pour une autre. De plus, le fait d'avoir des barreaux optiques interchangeables permet de ne stériliser que les barreaux optiques dans le cas où la poignée de préhension comprendrait des composants optiques et électroniques haut de gamme et/ou sensibles aux températures de stérilisation. Enfin, en changeant le barreau optique par un autre, il est possible de modifier l'optique de prise de vue. On pourrait prévoit, par exemple, de remplacer un premier barreau optique par un second barreau optique pour lequel la puissance de convergence des rayons lumineux est plus élevée ou pour lequel une lentille prismatique a été adjointe, côté invasif, pour faire varier l'axe de prise de vue d'un angle donné.

La poignée de préhension 30 est accouplée mécaniquement avec le barreau optique via des moyens d'accouplement mécanique réversible 6, 7. Plus précisément, le barreau optique 20 comprend à son extrémité proximale une partie filetée 6 complémentaire d'une ouverture filetée 7 ménagée dans la poignée de préhension 30 et permettant, par vissage mécanique, d'accoupler et de fixer solidairement le barreau optique 20 sur la poignée de préhension 30.

Les dimensions géométriques desdits moyens d'accouplement mécanique, tels que le diamètre et le pas de filetage, sont des caractéristiques que l'Homme du Métier est à même de définir en fonction des dimensions géométriques du barreau optique et du moyen de préhension souhaitées et permettant une fixation précise et aisée de ces deux éléments.

D'autres moyens d'accouplement peuvent être bien sûr être mis en œuvre sans sortir du cadre de l'invention, tels que des moyens d'accouplement par clipsage, par baïonnette (avantageux pour sa précision d'alignement mécanique et sa rapidité d'installation), par emboîtement, etc.

Un tel mode de réalisation permet d'offrir un endoscope ayant un montage simple.

Les deux modes de réalisation décrits ci-dessus sont destiné à l'endoscopie médicale, et notamment l'endoscopie oculaire. Il est clair toutefois qu'il peut aisément être adapté à de nombreuses autres applications, sans sortir du cadre de l'invention.

## Revendications

1. Dispositif endoscopique (1) comprenant une tête de guidage optique (2) destinée à être insérée, au moins en partie, dans un corps ou une cavité pour visualiser un objet donné inaccessible à l'œil nu, la tête de guidage optique étant constitué d'un barreau optique à base de verre organique et à gradient d'indice de réfraction, le dispositif endoscopique étant **caractérisé en ce que** le barreau optique comprend au moins un rail longitudinal (23) apte à recevoir une fibre optique d'éclairage (24) pour éclairer l'objet à visualiser à proximité de l'extrémité distale (22) du barreau optique.

2. Dispositif endoscopique selon la revendication 1, comprenant un capteur d'image (35) et dans lequel le barreau optique à gradient d'indice de réfraction comprend une extrémité proximale (21) et une extrémité distale (22) chacune traitée de façon former une image sur le capteur d'image (35) à partir de rayons lumineux issus de l'objet à visualiser.

3. Dispositif endoscopique selon la revendication 2, dans lequel au moins l'une desdites extrémité proximale (21) et distale (22) appartient à la liste comprenant :
- une extrémité plane ;
- une extrémité asphérique ;
- une extrémité en Fresnel diffractif.

4. Dispositif endoscopique selon l'une quelconque des revendications 1 à 3, comprend une lentille optique d'élargissement de champ de captation (25) agencée sur l'extrémité distale (22) du barreau optique.

5. Dispositif endoscopique selon l'une quelconque des revendications 1 à 4, comprenant un moyen de préhension (3) du dispositif endoscopique et dans lequel ledit moyen de préhension (3) et le barreau optique à gradient d'indice (2) forment un ensemble monobloc.

6. Dispositif endoscopique selon l'une quelconque des revendications 1 à 4, comprenant un moyen de préhension (3) du dispositif endoscopique, ledit moyen de préhension (3) étant accouplé mécaniquement avec le barreau optique à gradient d'indice (2) via des moyens d'accouplement mécanique réversible (7, 8).

7. Dispositif endoscopique selon la revendication 6, dans lequel les moyens d'accouplement mécanique réversible (7, 8) appartiennent au groupe comprenant :
- moyens d'accouplement par clipsage ;
- moyens d'accouplement par vissage ;
- moyens d'accouplement par emboîtement ;
- moyens d'accouplement à liaisons mâle et femelle ;
- moyens d'accouplement à baïonnette.

8. Dispositif endoscopique selon l'une quelconque des revendications 5 à 7, dans lequel le capteur d'image est un capteur d'image embarqué (32) dans le moyen de préhension (3).

9. Dispositif endoscopique selon la revendication 8, comprenant une optique d'adaptation de focale (34) agencée à proximité du capteur d'image embarqué (32), entre le barreau optique à gradient d'indice et le capteur d'image embarqué (32).

10. Dispositif endoscopique selon la revendication 9, dans lequel l'optique d'adaptation de focale (34) appartient au groupe comprenant :
- une lentille optique à focale fixe ;
- une série de lentilles amovible à focale variable ;
- une optique adaptative à base de liquide ou de cristal liquide.

11. Dispositif endoscopique selon l'une quelconque des revendications 5 à 10, dans lequel le moyen de préhension (3) comprend en outre au moins une source de lumière embarquée (34) et/ou une alimentation électrique autonome.

12. Dispositif endoscopique selon l'une quelconque des revendications 1 à 11, dans lequel le barreau optique à gradient d'indice (2) est recouvert d'un film de protection opaque.

## Patentansprüche

1. Endoskopische Vorrichtung (1), umfassend einen optischen Führungskopf (2), der dazu vorgesehen ist, zumindest zum Teil in einen Körper oder eine Höhle eingeführt zu werden, um ein gegebenes Objekt, das dem bloßen Auge unzugänglich ist, zu visualisieren, wobei der optische Führungskopf aus einem optischen Stab auf der Basis von organischem Glas und mit Brechungsindexgradient besteht, wobei die endoskopische Vorrichtung **dadurch gekennzeichnet ist, dass** der optische Stab mindestens eine Längsschiene (23) umfasst, die dazu geeignet ist, eine optische Beleuchtungsfaser (24) zum Beleuchten des zu visualisierenden Objekts in der Nähe des distalen Endes (22) des optischen Stabs aufzunehmen.

2. Endoskopische Vorrichtung nach Anspruch 1, umfassend einen Bildsensor (35), und wobei der optische Stab mit Brechungsindexgradient ein proximales Ende (21) und ein distales Ende (22) umfasst, die jeweils bearbeitet sind, um ein Bild auf dem Bildsensor (35) aus Lichtstrahlen, die von dem zu visualisierenden Objekt stammen, zu bilden.

3. Endoskopische Vorrichtung nach Anspruch 2, wobei mindestens eines von dem proximalen (21) und dem distalen Ende (22) zu der Liste gehört, umfassend:
- ein ebenes Ende;
- ein asphärisches Ende;
- ein Fresnel-Beugungsende.

4. Endoskopische Vorrichtung nach einem der Ansprüche 1 bis 3, die eine optische Linse zur Erweiterung des Erfassungsfelds (25) umfasst, die am distalen Ende (22) des optischen Stabs eingerichtet ist.

5. Endoskopische Vorrichtung nach einem der Ansprüche 1 bis 4, umfassend ein Greifmittel (3) der endoskopischen Vorrichtung, und wobei das Greifmittel (3) und der optische Stab mit Brechungsindexgradient (2) eine einteilige Einheit bilden.

6. Endoskopische Vorrichtung nach einem der Ansprüche 1 bis 4, umfassend ein Greifmittel (3) der endoskopischen Vorrichtung, wobei das Greifmittel (3) mittels umkehrbaren mechanischen Kopplungsmitteln (7, 8) mechanisch mit dem optischen Stab mit Brechungsindexgradient (2) gekoppelt ist.

7. Endoskopische Vorrichtung nach Anspruch 6, wobei die umkehrbaren mechanischen Kopplungsmittel (7, 8) zu der Gruppe gehören, umfassend:
- Schnappkopplungsmittel;
- Schraubkopplungsmittel;
- Falzkopplungsmittel;
- Stecker- und Buchsenkopplungsmittel;
- Bajonettkopplungsmittel.

8. Endoskopische Vorrichtung nach einem der Ansprüche 5 bis 7, wobei der Bildsensor ein in dem Greifmittel (3) eingebetteter Bildsensor (32) ist.

9. Endoskopische Vorrichtung nach Anspruch 8, umfassend eine optische Brennweitenanpassung (34), die in der Nähe des eingebetteten Bildsensors (32) zwischen dem optischen Stab mit Brechungsindexgradient und dem eingebetteten Bildsensor (32) eingerichtet ist.

10. Endoskopische Vorrichtung nach Anspruch 9, wobei die optische Brennweitenanpassung (34) zu der Gruppe gehört, umfassend:
- eine optische Linse mit fester Brennweite;
- eine Reihe von herausnehmbaren Linsen mit variabler Brennweite;
- eine adaptive Optik auf Flüssigkeits- oder Flüssigkristallbasis.

11. Endoskopische Vorrichtung nach einem der Ansprüche 5 bis 10, wobei das Greifmittel (3) weiterhin mindestens eine eingebettete Lichtquelle (34) und/oder eine unabhängige Stromversorgung umfasst.

12. Endoskopische Vorrichtung nach einem der Ansprüche 1 bis 11, wobei der optische Stab mit Brechungsindexgradient (2) mit einer opaken Schutzfolie bedeckt ist.

## Claims

1. Endoscopic device (1) comprising an optical guide head (2) intended to be inserted, at least in part, into a body or a cavity in order to view a given object that is inaccessible to the naked eye, the optical guide head is constituted by an optical bar made out of organic glass and with refraction index gradient, the endoscopic device being **characterized in that** the optical bar comprises at least one longitudinal rail (23) capable of receiving an optical illumination fiber (24) to illuminate the object to be viewed in proximity to the distal end (22) of the optical bar.

2. Endoscopic device according to claim 1, comprising an image sensor (35) and wherein the optical bar with refraction index gradient comprises a proximal end (21) and a distal end (32), each treated so as to form an image on the image sensor (35) from light rays coming from the object to be viewed.

3. Endoscopic device according to claim 2, wherein at least one of said ends, namely said proximal end (21) and said distal end (22), belongs to the list comprising:
- a flat end;
- an aspherical end;
- a diffractive Fresnel end.

4. Endoscopic device according to any one of the claims 1 to 3, comprising a sensing field expanding optical lens (25) laid out at the distal end of the optical bar.

5. Endoscopic device according to any one of the claims 1 to 4, comprising a means (3) for gripping the endoscopic device and wherein said means (3) for gripping and the optical bar with index gradient (2) form a single-piece unit.

6. Endoscopic device according to any one of the claims 1 to 4, comprising a means (3) for gripping the endoscopic device, said means (3) for gripping being mechanically coupled with the optical bar with index gradient (2) via reversible mechanical coupling means (7, 8).

7. Endoscopic device according to claim 6, wherein reversible mechanical coupling means (7, 8) belong to the group comprising:
- clip-on coupling means;
- screw-in coupling means;
- plug-in coupling means;
- male/female linkage coupling means;
- bayonet coupling means.

8. Endoscopic device according to any one of the claims 5 to 7, wherein the image sensor is an image sensor (32) embedded in the means (3) for gripping.

9. Endoscopic device according to claim 8, comprising a focal-distance adapting optical system (34) positioned in proximity to the embedded image sensor (34), between the optical bar with index gradient and the embedded image sensor (32).

10. Endoscopic device according to claim 9, wherein the focal-distance adapting optical system (34) belongs to the group comprising:
- an optical lens with fixed focal distance;
- a series of detachable lenses with variable focal distance;
- a liquid-based or liquid-crystal-based adaptive optical system.

11. Endoscopic device according to any one of the claims 5 to 10, wherein the means (3) for gripping furthermore comprise at least one embedded light source (34) and/or an autonomous electrical supply.

12. Endoscopic device according to any one of the claims 1 to 11, wherein the optical bar with index gradient (2) is coated with an opaque protective film.
